# EUROPEAN PATENT SPECIFICATION

(11) **EP 3 317 397 B1**
(45) Date of publication and mention of the grant of the patent: **25.09.2019**
(21) Application number: 16736040.3
(22) Date of filing: 01.07.2016
(51) Int. Cl.: A61K 35/15, A61K 35/28

(54) **SECRETOMES AND METHOD FOR PRODUCING SECRETOMES**
SEKRETOME UND VERFAHREN ZUR HERSTELLUNG VON SEKRETOMEN
SÉCRÉTOMES ET PROCÉDÉ DE PRODUCTION DE SÉCRÉTOMES

(30) Priority: 02.07.2015 CH 9562015
(43) Date of publication of application: 09.05.2018
(73) Proprietor: Med Cell Bahamas Ltd., Nassau (BS)
(72) Inventor: KELLNER, Steven, 9542 Münchwilen (CH)
(74) Representative: Rentsch Partner AG
(86) International application number: PCT/EP2016/065473
(87) International publication number: WO 2017/001649

(56) References cited:
- EP-A1- 2 607 478
- WO-A1-2013/150303
- WO-A1-2016/082882
- WO-A1-2016/083500
- YULIANG FENG ET AL: "Ischemic Preconditioning Potentiates the Protective Effect of Stem Cells through Secretion of Exosomes by Targeting Mecp2 via miR-22", PLOS ONE, vol. 9, no. 2, 18 February 2014 (2014-02-18), page e88685, XP055293659, DOI: 10.1371/journal.pone.0088685
- CLARA GALLINA ET AL: "A New Paradigm in Cardiac Regeneration: The Mesenchymal Stem Cell Secretome", STEM CELLS INTERNATIONAL, vol. 262, no. 19, 1 January 2015 (2015-01-01), pages 9412-10, XP055293175, US ISSN: 1687-966X, DOI: 10.1002/jcp.20959
- SERENA RUBINA BAGLIO ET AL: "Human bone marrow- and adipose-mesenchymal stem cells secrete exosomes enriched in distinctive miRNA and tRNA species", STEM CELL RESEARCH & THERAPY, vol. 87, no. 1, 1 July 2015 (2015-07-01), page 257, XP055293655, London, UK ISSN: 1757-6512, DOI: 10.1186/s13287-015-0116-z
- CHING-PING CHANG ET AL: "Hypoxic preconditioning enhances the therapeutic potential of the secretome from cultured human mesenchymal stem cells in experimental traumatic brain injury", CLINICAL SCIENCE, vol. 8, no. 3, 5 October 2012 (2012-10-05) , pages 4A1.1-176, XP055161098, ISSN: 0143-5221, DOI: 10.1016/j.neulet.2008.03.096
- LEI CHEN ET AL: "Conditioned Medium from Hypoxic Bone Marrow-Derived Mesenchymal Stem Cells Enhances Wound Healing in Mice", PLOS ONE, vol. 9, no. 4, 29 April 2014 (2014-04-29), page e96161, XP055161042, DOI: 10.1371/journal.pone.0096161
- SUDHIRH RANGANATH ET AL: "Harnessing the Mesenchymal Stem Cell Secretome for the Treatment of Cardiovascular Disease", CELL STEM CELL, vol. 10, no. 3, 2 March 2010 (2010-03-02), pages 244-258, XP028402789, ISSN: 1934-5909, DOI: 10.1016/J.STEM.2012.02.005 [retrieved on 2012-02-14]
- KUPCOVA SKALNIKOVA HELENA ED - SALGADO ANTONIO ET AL: "Proteomic techniques for characterisation of mesenchymal stem cell secretome", BIOCHIMIE, vol. 95, no. 12, 20 July 2013 (2013-07-20) , pages 2196-2211, XP028768034, ISSN: 0300-9084, DOI: 10.1016/J.BIOCHI.2013.07.015
- HYUN OK KIM ET AL: "Mesenchymal stem cell-derived secretome and microvesicles as a cell-free therapeutics for neurodegenerative disorders", TISSUE ENGINEERING AND REGENERATIVE MEDICINE, vol. 10, no. 3, 25 May 2013 (2013-05-25), pages 93-101, XP055161063, ISSN: 1738-2696, DOI: 10.1007/s13770-013-0010-7
- WANG J A ET AL: "Anoxic preconditioning: A way to enhance the cardioprotection of mesenchymal stem cells", INTERNATIONAL JOURNAL OF CARDIOLOGY, ELSEVIER SCIENCE PUBLISHERS, AMSTERDAM, NL, vol. 133, no. 3, 17 April 2009 (2009-04-17), pages 410-412, XP026080488, ISSN: 0167-5273, DOI: 10.1016/J.IJCARD.2007.11.096 [retrieved on 2008-01-29]
- GÁLVEZ-MARTÍN PATRICIA ET AL: "Study of the stability of packaging and storage conditions of human mesenchymal stem cell for intra-arterial clinical application in patient with critical limb ischemia", EUROPEAN JOURNAL OF PHARMACEUTICS AND BIOPHARMACEUTICS, vol. 86, no. 3, 14 November 2013 (2013-11-14), pages 459-468, XP028845357, ISSN: 0939-6411, DOI: 10.1016/J.EJPB.2013.11.002
- I. J. DE KOK ET AL: "Effect of Vitamin D Pretreatment of Human Mesenchymal Stem Cells on Ectopic Bone Formation", JOURNAL OF ORAL IMPLANTOLOGY, vol. 32, no. 3, 1 June 2006 (2006-06-01), pages 103-109, XP055293081, US ISSN: 0160-6972, DOI: 10.1563/760.1
- ZORAN IVANOVIC ET AL: "Primitive human HPCs are better maintained and expanded in vitro at 1 percent oxygen than at 20 percent", TRANSFUSION, vol. 40, no. 12, 1 December 2000 (2000-12-01), pages 1482-1488, XP055161089, ISSN: 0041-1132, DOI: 10.1046/j.1537-2995.2000.40121482.x

## Description

### Field of the invention

The present invention relates to the fields of secretomes. More particularly, the invention relates to a method of deriving secretomes from mesenchymal stem cells.

### Background

Mesenchymal stem cells, or MSCs, are multipotent stromal cells which have the potential to differentiate into a variety of mesenchymal cell types of the adipocytic, chondrocytic and osteocytic lineages, including: osteoblasts, chondrocytes, neurons, muscle cells and adipocytes. This potential has been documented in specific cells and tissues in vivo and in vitro. MSCs are distributed in mammals all over the body and are responsible for regeneration. Commonly used tissues for the isolation of MSC are bone marrow, umbilical cord, cord lining and - increasingly - adipose tissue, which has a superior amount of MSCs.

Dendritic cells, or DCs, are antigen-presenting cells (APCs) which play a critical role in the regulation of the adaptive immune response. Dendritic cells are unique APCs and have been referred to as "professional" APCs, since the principal function of DCs is to present antigens, and because only DCs have the ability to induce a primary immune response in resting naive T-lymphocytes. To perform this function, DCs are capable of capturing antigens, processing them, and presenting them on the cell surface along with appropriate co-stimulation molecules. DCs also play a role in the maintenance of B-cell function and recall responses. Thus, DCs are critical in the establishment of immunological memory. The function of DCs falls broadly into three categories, each of which involve antigen presentation. The first category of DCs function is antigen presentation and activation of T-cells. The second category of DC function is not as well established, but it has been suggested that a different class of DCs exist with the function of inducing and maintaining immune tolerance. The third category of DCs, known as follicular DCs, appear to work to maintain immune memory in tandem with B-cells. DCs can be generated by transforming peripheral blood monocytes.

All cells communicate and exchange information by different ways, including the secretion of soluble factors, the cell-to-cell adhesion contact, and the intercellular exchange of organelles. The secretome of mesenchymal stem cells includes paracrine substances, exosomes and microvesicles. Over 150 paracrine substances, also called cytokines and chemokines, can be released by mesenchymal stem cells. Two distinct populations of vesicles with peculiar membrane structure, mechanism of production, pathophysiological relevance, and different size have been described: exosomes and microvesicles. Microvesicles and exosomes contain biomolecules, including messenger RNA and micro RNA. Exosomes and the whole secretome have been used for therapies in regenerative medicine to treat various illnesses such as osteoarthritis, cardiovascular diseases, neurological diseases, pulmonary diseases, diabetes and many more.

In the known therapies success rate and duration has been very variable, mainly due to the insufficient amount of proteins and RNA used in the studies.

The secretome of both MSCs and DCs consists of various proteins, RNAs and extracellular vesicles, such as, but not limited to, exosomes and microvesicles.

### 1. Introduction to exosomes and microvesicles

Cells communicate and exchange information by different ways, including the secretion of soluble factors, the cell-to-cell adhesion contact, and the intercellular exchange of organelles through nanotubular structures^{[1]}. Recent studies have proposed that cell-derived small circular membrane vesicles, called exosomes and microvesicles, represent an additional mechanism of cell-to-cell communication by transfer of membrane components as well as the cytoplasmic content ^{[2,3,4,5]}. Two distinct populations of vesicles with peculiar membrane structure, mechanism of production, pathophysiological relevance, and different size have been described. Membrane fragments of 30-100nm diameter derived from the endosomal membrane compartment afterfusion of secretory granules with the plasma membrane are defined as exosomes. Once released, exosomes bind the recipient cells through receptor-ligand interactions or fuse with the target cell membrane transferring membrane components, including cell receptors^{[2]}, and discharging the portion of cytosol segregated within their lumen into the cytoplasm of recipient cells^{[6]}. The molecular cargo content of exosomes derives from active packaging of certain nucleic acid species leading to the presence of mRNAs in exosomes that are not found in donor cells^{[7]}. Furthermore, relatively large microvesicles (100nm - 1 mm diameter) are formed from the surface membrane of activated cells in a calcium and calpain dependent manner following a disordered function of phospholipid transporters that results in the budding of altered membrane that exposes phosphatidylserine in the outer leaflet. Microvesicles and exosomes contain biomolecules, including mRNA and microRNA (miRNA), packaged in a random process and their release is considered an expression of a pathological process in place. Molecular transfer from microvesicles and exosomes contributes to changes in the maturation and differentiation of target cells as for example microvesicles and exosomes released by endothelial progenitor cells trigger neo-an-giogenesis in endothelial cells^{[8]}.

### 2. Introduction to paracrine substances

MSCs secrete numerous growth factors and cytokines. A typical MSC secretion profile comprises growth factors, cytokines, ECM proteases, hormones, and lipid mediators. Paracrine signaling is a form of cell-cell communication in which a cell produces a signal to induce changes in nearby cells, altering the behavior or differentiation of those cells. Signaling molecules known as paracrine factors diffuse over a relatively short distance (local action), as opposed to endocrine factors (hormones which travel considerably longer distances via the circulatory system), juxtacrine interactions, and autocrine signaling. Cells that produce paracrine factors secrete them into the immediate extracellular environment. Factors then travel to nearby cells in which the gradient of factor received determines the outcome. However, the exact distance that paracrine factors can travel is not certain. Although paracrine signaling elicits a diverse array of responses in the induced cells, most paracrine factors utilize a relatively streamlined set of receptors and pathways. In fact, different organs in the body -even between different species - are known to utilize a similar sets of paracrine factors in differential development. The highly conserved receptors and pathways can be organized into four major families based on similar structures: Fibroblast growth factor (FGF) family, Hedgehog family, Wnt family, and TGF-β superfamily. Binding of a paracrine factor to its respective receptor initiates signal transduction cascades, eliciting different responses.

### 3. Introduction to manipulation and preconditioning of MSCs

Due to the potential of MSCs and secretomes obtained therefrom for therapeutic applications, various strategies have been proposed to obtain increased production or secretion of paracrine factors as described above.

Previous studies suggested that physiological preconditioning such as a hypoxic condition promotes self-renewal of undifferentiated mesenchymal stem cells and enhances therapeutic potential. However, neither of these studies disclose a production of clinical grade secretomes under anoxic preconditioning or a preconditioning with Ringer's lactate or Vitamin D, let alone the effect of these preconditioning methods on the content of the obtained secretomes (see e.g. Feng, et al. Plos One, vol 9, p. e88685, 2014; Gallina et al, Stem Cells International, vol. 262, p 9412, 2015; Baglio et al, Stem Cell Research & Therapy, vol. 87, p. 257, 2015; Chang et al, Clinical Science, vol. 8. p. 4A1.1-176. 2012; Chen et al, Plos One, vol. 9. p. e96161, 2014; Ranganath et al, Cell Stem Cell, vol. 10, p. 244, 2010; Skalnikova, Biochimie, vol. 95, p. 2196, 2013: EP2607478A1 ; Kim et al. Tissue Engineering and Regenerative Medicine, vol. 10. p. 93, 2013; WO 2013/150303 A1; Wang et al, Int. J. of Cardiology, vol. 133, p. 410, 2009). Hypoxic exposure activates several signal transduction pathways including hypoxia inducible factor (HIF), a master transcription factor that regulates the expression of hundreds of genes to promote cellular adaptation to the hypoxic condition. HIF-1a is a pivotal signaling molecule for hypoxia-mediated upregulation of bFGF and hepatocyte growth factor (HGF) secretion, whereas HIF-2a is a key signaling molecule for hypoxia-mediated upregulation of VEGF secretion from MSCs. HIF-1a is widely expressed in almost all tissues, whereas the expression of HIF-2a is restricted to certain cell types such as vascular endothelial cells.

Different strategies have focussed on genetic manipulation of MSCs, whereby MSCs have been engineered with transgenes for conditional gene expression with the aim of improving cell survival and controlling the MSC secretome post-transplantation.

Yet other studies have aimed at controlling MSC paracrine secretion through molecular preconditioning using compounds ranging from proteins such as cytokines, chemokines and growth factors to small molecule libraries.

Yet, despite promising results, neither strategy has been able to adress all requirements and there remains still a need for improved methods for the production of secretomes.

### SUMMARY OF THE INVENTION

According to a first aspect of the present invention, a method of producing a secretome secreted by a mesenchymal stem cell comprising at least one biological property of a mesenchymal stem cell is provided. According to the present invention, the cells are treated for at least one period with Ringer's lactate solution and/or Vitamin D. Optionally, the cells may also be kept during at least one further period of at least 5 minutes under anoxic conditions at an Oxygen concentration below 1 %, preferably below 0.5 %. Surprisingly the treatment with Ringer's lactate solution and/or Vitamin D had a significant positive influence on the amount and quality of exosomes, microvesicles and/or paracrine substances secreted by mesenchymal stem cells or secreted by the dendritic cells.

The secretome or secretome vesicles or paracrine secretome substances secreted by a mesenchymal stem cell comprising at least one biological property of a mesenchymal stem cell produced according to the present invention or
a pharmaceutical composition thereof together with a pharmaceutically acceptable carrier, excipient or diluent may be suitable for use in a method of treating a disease, in particular for use in a method of autogenous medical treatment of a disease or autogenous cosmetic treatment in an individual (which acted as donor for the mesenchymal stem cell or the dendritic cell used for producing the secretome or secretome vesicle or paracrine substance).

The disease may be selected from the group consisting of: osteoarthritis, cardiovascular diseases, lung diseases, liver diseases, neurodegenerative diseases including multiple sclerosis, Parkinson, Alzheimer and Graft-versus-host-disease (GVHD) or Crohn's disease.

The secretome or secretome vesicle or paracrine secretome substance may be used to aid wound healing, scar reduction or cartilage- or bone-formation.

"Ringer's lactate solution" typically refers to a solution of about 0.86 gram sodium chloride (NaCl), 0.03 gram potassium chloride (KCl), and 0.033 gram calcium chloride (CaCl) in purified water.

"Vitamin D" refers to any active form of vitamin D, i.e. the Ict,25- dihydroxyvitamin D (Ia,25-dihydroxycholecalciferol, calcitriol) or its metabolites or partially active precursors, such as 25-hydroxyvitamin D3 (25-hydroxycholecalciferol, calcifediol), vitamin D3 (cholecalciferol), vitamin D2 (ergocalciferol), 1-a-hydroxycholecalciferol (alfacalcidol), 25-hydroxyvitamin D3 3-sulphate, dihydroxytachisterol, doxercalciferol, provitamin D3 (7- dehydrocholesterol) and vitamin D derivatives or analogs such as, for example, falecalcitriol, maxacalcitol and paricalcitol.

"Oxic conditions" refers in the present description to ambient atmospheric oxygen tension of approximately 5-21 % O₂ (volume:volume; 1'013 hPa; 50'000 - 210'000 parts per million Oxygen).

"Hypoxic conditions" refers to oxygen tensions between approximately 1 -5 % O₂ (10'000 - 50'000 ppm Oxygen).

"Anoxic conditions" refers to oxygen tensions below approximately 1 % O₂ (10'000 ppm Oxygen), preferably below 0.5 % O₂ (5'000 ppm Oxygen).

The practice of the present invention will employ, unless otherwise indicated, conventional techniques of chemistry, molecular biology, microbiology, recombinant DNA and immunology, which are within the capabilities and the general knowledge of a person of ordinary skill in the art. Such techniques are explained in the literature. See, for example [13], [14] and [15].

### BRIEF DESCRIPTION OF THE FIGURES

- Figure 1: shows a comparison of messenger RNA content (mRNA, column A) and micro RNA content (miRNA, column B) in ng/ul of sectretome produced in the presence of either PBS or Ringer's lactate (RL).
- Figure 2: shows a comparison of messenger RNA content (mRNA, column A) and micro RNA content (miRNA, column B) in ng/ul of sectretome produced of cells pretreated with vitamin D in different concentrations (DN: no Vitamin D, D1: 600 IU Vitamin D, D2: 6000 IU Vitamin D).
- Figure 3: shows in a bar graph the measured amounts (ocular density (OD) measurement in photospectrometer) of neprilysin and HGF produced of cells pretreated with vitamin D in different concentrations (DN: no Vitamin D, D1: 600 IU Vitamin D, D2: 6000 IU Vitamin D).

### Detailed description of the invention

Preferably MSCs are thawed and cultivated and DCSs are thawed in cell culture flasks with a special cell medium (nutrient). According to a first preferred method, the cells are then detached and washed three times in warm phosphate buffered saline (PBS) or other saline solutions. According to a second preferred method the cells are washed directly in the cell culture flasks without detachment.

After washing according to either one of the above mentioned systems, 2 to 15 ml of warm Ringer's lactate solution (or PBS solution as control) is added and the cells are kept at oxic conditions at room temperature or at 37°C for a total of between 5 and 24 hours.

Alternatively, after washing according to either one of the above mentioned systems, 2 to 15 ml of warm Ringer's lactate solution or PBS solution containing Vitamin D (up to 12000 IU, for example between 600 and 6000 IU) is added and the cells are kept at oxic conditions at room temperature or at 37°C for a total of between 5 and 24 hours.

Optionally, the cells may be kept in addition for at least one period of at least 5 minutes under anoxic conditions before leaving them at oxic conditions. The duration of the period under anoxic conditions lies preferably in a range of between 5-120 minutes. According to further preferred embodiments of the present invention the cells are exposed to more than one period under anoxic conditions, preferably the cells are exposed to 2 to 4 periods of anoxic conditions each followed by a subsequent period of keeping the cells under oxic conditions. Each period under anoxic conditions followed by a period under oxic conditions is called a cycle. During the anoxic cycle the oxygen concentration is below 1 %, preferably below 0.5 %.

The secretome is then harvested for example by simple aspiration of PBS. The secretome is then preferably centrifuged to discard cells and afterwards microfiltrated to include or exclude various components of the secretome.

Compared to previously known systems exposing MSC cells to stress conditions the novel production method results in a high increase in the amount of RNA in the secretome.

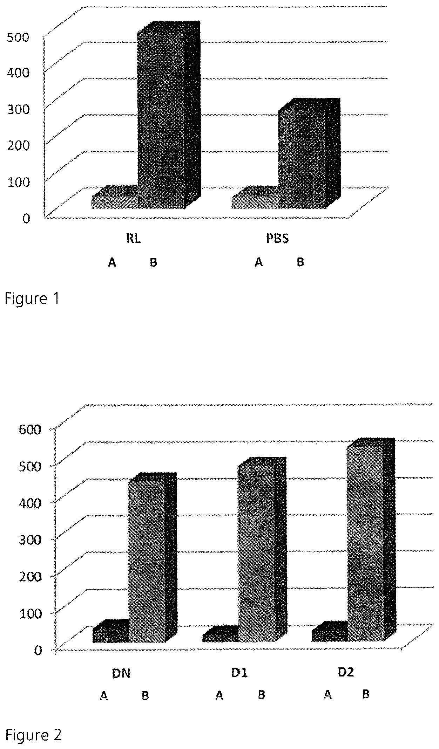

In particular, the high amount of miR in the secretome after the treatment with Ringer's lactate and/or Vitamin D is advantageous (see Figures 1 and 2). For example, miR-22 which can be used in a method of treating cardiovascular diseases, miR-133b for neurological diseases, miR-146 for wound healing, miR-204 for pulmonary diseases. Thereby the present invention allows the person skilled in the art to produce secretome specifically for certain diseases.

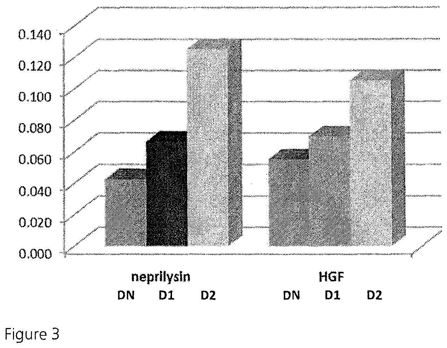

Moreover, high increases were observed in factors believed to be beneficial in the treatment of cognitive impairment, such as neprilysin, or the multifunctional growth factor HGF (see Figure 3)

In particular, clinical grade secretome for therapies in regenerative medicine for a host of diseases such as osteoarthritis, cardiovascular diseases, lung diseases, liver diseases, neurodegenerative diseases including multiple sclerosis, Parkinson, Alzheimer, GVHD, Crohn's disease and many more.

All of the above said is not only true for secretome, but also for secretome vesicles, in particular for exosomes and/or microvesicles, and for paracrine secretome substances. The person skilled in the art knows how to purify, extract etc. said vesicles and substances from the whole secretome for example by utilizing the teachings from [16] and [17] which are incorporated herein by reference.

### Example 1 (Pretreatment with Ringer's lactate solution)

According to a first example secretome is produced by the following steps:
1.1. Fill a precoated T75 culture flask with 15 ml of SMP alpha minus stem cell medium and transfer into incubator for 30-120 minutes. Then spray culture flask with 70% IPA and transfer to laminar air flow.
1.2. Thaw a cryopreserved vial with approximately 1 mio MSC by removing it from the liquid nitrogen storage tank and transfer it immediately into the warm water bath preheated to 37-40 °C for 1-4 minutes. Remove vial from bath immediately after last bit of solid ice has been thawed
1.3. Spray vial with 70% alcohol and transfer to laminar air flow. Pipette MSCs with 2ml serological pipette into the ready culture flask and swirl gently and transfer to incubator (5% CO₂ and 37 °C)
1.4. Pipette 15 ml of SMP alpha minus stem cell medium into a 50 ml centrifuge tube and transfer to water bath for at least 30 minutes.
1.5. Change stem cell media 2-8 hours after transfer of vial into cell culture flask. Verify that at least 90% of the cells have settled to the plastic floor.
1.6. Leave culture flask in the incubator for 1 to 3 days until a cell confluence of 75%-90% has been achieved.
1.7. Transfer flask to laminar air flow bench after spraying with 70% IPA and remove supernatant. Wash Flask with warm Dulbecco's PBS or similar three times with 10 ml each carefully.
1.8. Cover the cells with either 7 ml prewarmed Ringer's lactate or 7 ml Dulbecco's PBS solution (or other saline solution as control) and incubate at room temperature for a specified time.
1.9. Remove the Ringer's lactate or PBS solution under sterile conditions under the laminar air flow and centrifuge at 300 G for 3 minutes to remove detached stem cells. If only Exosomes are needed for the therapy, filtrate the Ringer's lactate or PBS solution afterwards through a 0.45 µm syringe filter to remove microvesicles. Microvesicles can be retrieved per reverse washing of the filter.
1.1 1. Transfer Ringer's lactate or PBS solution into a glass vial for immediate application or store at -800 °C in a RNAase free plastic centrifuge tube.
1.12. Add 15 ml of SMP alpha minus stem cell medium to the culture flask and return it to the incubator.
1.13. Leave in incubator, with media changes every 2-3 days if necessary until cells have regrown to 80% confluence before repeating the harvesting procedure or freezing them according the standard procedures in liquid nitrogen.

### Example 2 (Pretreatment with Vitamin D)

According to a second example secretome is produced by the following steps:
The prodecure of Example 1 is followed with the exception that in step the cells are placed into flasks containing (i) 15ml Ringer's lactate (or saline) solution (called DN) or (ii) 15ml Ringer's lactate (or saline) solution containing 600 IU Vitamin D (called D1) or (iii) 15ml Ringer's lactate (or saline) solution containing 6000 IU Vitamin D (called D2) and incubated at room temperature overnight.

### Example 3: Optional steps:

Optionally the cells can be subjected in addition to anoxic conditions as follows:
1.8.a After covering the cells with the saline solution of choice (optionally in the presence of Vitamin D) (step 1.8), prepare the AnaerobJar Oxoid system by spraying with 70% IPA and leaving to dry in the laminar air flow.
1.8.b. Place the culture flask into the lying Anaerobjar, insert the oxygen capture sachet and close the lid of the jar. Leave for 1 hour in the laminar air flow bench at room temperature, then open the jar carefully and place flask in the CO₂ incubator or at room temperature in the bench for 4 hours. Instead of the anaerob jars a CO₂/O₂ incubator or an alternative device for culturing the cells under anoxic conditions in an enclosed volume can be used (e.g. an Hypoxia Incubator Chamber produced by STEMCELL Technologies SARL, 40 Rue des Berges, Miniparc Polytec, Bâtiment Sirocco, 38000 Grenoble, France) or other newly designed anoxia systems.

### Material und Devices

1 Reagents and Solvents
   (1) SMP alpha minus
   (2) PBS Dulbecco or similar w/o Ca/Mg
   (3) Ringer's lactate solution
2 Devices
   (1) CO2 Incubator / CO2/O2 incubator
   (2) Laminar flow bench
   (3) Centrifuge
   (4) Water bath 35 ± 4°C
   (5) Pipetting aid and serological pipettes
   (6) Inverted Microscope Bresser
   (7) Anaerob Jar or similar system
   (8) Precoated culture flasks or self coated flasks
   (9) Centrifuge tubes, RNAase free

Ther person skilled in the art can, based on the technical information provided above and utilizing his technical knowledge, upscale the production methods according to the examples provided above without inventive activity.

### References

1. Rustom A, R Saffrich, I Markovic, P Walther and HH Gerdes. (2004). Nanotubular highways for intercellular organelle transport. Science 303:1007-1010.
2. Ratajczak J, M Wysoczynski, F Hayek, A Janowska-Wieczorek and MZ Ratajczak. (2006). Membrane-derived microvesicles: important and underap-preciated mediators of cell-to-cell communication. Leukemia 20:1487-1495.
3. Camussi G, MC Deregibus, S Bruno, V Cantaluppi and L Biancone. (2010). Exosomes/microvesicles as a mechanism of cell-to-cell communication. Kidney Int 78:838-848.
4. Denzer K, MJ Kleijmeer, HF Heijnen, W Stoorvogel and HJ Geuze. (2000). Exosome: from internal vesicle of the multivesicular body to intercellular signaling device. J Cell Sci 113 Pt 1 9:3365-3374.
5. Fevrier B and G Raposo. (2004). Exosomes: endosomal-derived vesicles shipping extracellular messages. Curr Opin Cell Biol 16:415-421 .
6. Cocucci E, G Racchetti and J Meldolesi. (2009). Shedding microvesicles: artefacts no more. Trends Cell Biol 19:43-51 .
7. Valadi H, K Ekstrom, A Bossios, M Sjostrand, JJ Lee and JO Lotvall. (2007). Exosome-mediated transfer of mRNAs and microRNAs is a novel mechanism of genetic exchange between cells. Nat Cell Biol 9:654-659.
8. Deregibus MC, V Cantaluppi, R Calogero, M Lo Iacono, C Tetta, L Biancone, S Bruno, B Bussolati and G Camussi. (2007). Endothelial progenitor cell derived microvesicles activate an angiogenic program in endothelial cells by a horizontal transfer of mRNA. Blood 110:2440-2448.
9. Bo Yu, Xiaomin Zhang and Xiaorong Li. (2014). Exosomes Derived from Mesenchymal Stem Cells. Int. J. Mol. Sci. 1 5, 4142-41 57.
10. Eun Kyoung Jun et al. (2014) Hypoxic Conditioned Medium from Human Amniotic Fluid-Derived Mesenchymal Stem Cells Accelerates Skin Wound Healing through TGF-β/SMAD2 and PI3K/Akt Pathways. Int. J. Mol. Sci. 1 5, 605-628
11. Yi Lee et al. (2012). Exosomes and microvesicles: extracellular vesicles for genetic information transfer and gene therapy. Human Molecular Genetics. Vol. 21 , Review Issue 1
12. Imhof Alexander, Heinzer Ivo. (1996). Continuous Monitoring of Oxygen Concentrations in Several Systems for Cultivation of Anaerobic Bacteria. JOURNAL OF CLINICAL MICROBIOLOGY, 1646-1648
13. Gstraunthaler Gerhard, Lindl Toni. Zell- und Gewebekultur: Allgemeine Grundlagen und spezielle Anwendungen. 7. Ed. 2013, XIII, Heidelberg: Spektrum Akademischer Verlag
14. Øystein Bruserud (Author), Astrid Englezou (Editor). (2012). Cell culture technology: Recent advances and future prospects (Euroscicon Meeting Reports Book 1). Honnao Publishing
15. Lanza Robert P, Klimanskaya Irina (Editors). (2009). Essential Stem Cell Methods (Reliable Lab Solutions). Academic Press
16. Graça Raposo, Willem Stoorvogel. (2013). Extracellular vesicles: Exosomes, microvesicles, and friends. J. Cell Biol., Vol. 200 No. 4 373-383
17. Tauro BJ1, Greening DW, Mathias RA, Ji H, Mathivanan S, Scott AM, Simpson RJ. (2012). Comparison of ultracentrifugation, density gradient separation, and immunoaffinity capture methods for isolating human colon cancer cell line LIM1863-derived exosomes. Methods. 56(2):293-304. doi: 10.1016/j.ymeth.2012.01.002. Epub 2012 Jan 21 .

## Claims

1. A method of producing a secretome secreted by a mesenchymal stem cell comprising at least one biological property of a mesenchymal stem cell wherein the method comprises at least one period of treating the cells with Ringer's lactate solution and/or Vitamin D.

2. The method according to claim 1, **characterized in that** the method further comprises at least one period of keeping the cells at least 5 minutes under anoxic conditions.

3. The method according to claim 2, **characterized in that** the period of keeping the cells under anoxic conditions is followed by a period of keeping the cells under oxic conditions.

4. The method according to claim 3, **characterized in that** a cycle comprising a period of keeping the cells under anoxic conditions and a subsequent period of keeping the cells under oxic conditions is performed more than one time, preferably 2 to 4 times.

5. The method according to any of claims 2 to 4, **characterized in that** the Oxygen concentration during the anoxic step is below 1%, preferably below 0.5 %.

6. The method according to any of claims 2 to 5, **characterized in that** exosomes, microvesicles and/or paracrine secretome substances are isolated separately or in groups from the secretome.

7. The method according to any of claims 2 to 6, wherein the cells are kept under anoxic conditions in an AnaerobJar, a CO₂/O₂ incubator or another device for culturing the cells under anoxic conditions.

## Patentansprüche

1. Verfahren zur Herstellung eines Sekretoms, das von einer mesenchymalen Stammzelle abgesondert wird, umfassend mindestens eine biologische Eigenschaft einer mesenchymalen Stammzelle, wobei das Verfahren mindestens einen Zeitraum einer Behandlung der Zellen mit Ringer-Lactatlösung und/oder Vitamin D umfasst.

2. Verfahren nach Anspruch 1, **dadurch gekennzeichnet, dass** das Verfahren ferner mindestens einen Zeitraum von mindestens 5 Minuten des Haltens der Zellen unter anoxischen Bedingungen umfasst.

3. Verfahren nach Anspruch 2, **dadurch gekennzeichnet, dass** auf den Zeitraum des Haltens der Zellen unter anoxischen Bedingungen ein Zeitraum des Haltens der Zellen unter oxischen Bedingungen folgt.

4. Verfahren nach Anspruch 3, **dadurch gekennzeichnet, dass** ein Zyklus, umfassend einen Zeitraum des Haltens der Zellen unter anoxischen Bedingungen und einen darauffolgenden Zeitraum des Haltens der Zellen unter oxischen Bedingungen, mehr als einmal durchgeführt wird, vorzugsweise 2 bis 4 Mal.

5. Verfahren nach einem der Ansprüche 2 bis 4, **dadurch gekennzeichnet, dass** die Sauerstoffkonzentration während des anoxischen Schritts weniger als 1 % beträgt, vorzugsweise weniger als 0,5 %.

6. Verfahren nach einem der Ansprüche 2 bis 5, **dadurch gekennzeichnet, dass** Exosome, Mikrovesikel und/oder parakrine Sekretomsubstanzen getrennt oder in Gruppen aus dem Sekretom isoliert werden.

7. Verfahren nach einem der Ansprüche 2 bis 6, wobei die Zellen in einem AnaerobJar, einem CO₂/O₂-Inkubator oder einer anderen Vorrichtung zum Züchten der Zellen unter anoxischen Bedingungen gehalten werden.

## Revendications

1. Procédé de production d'un sécrétome sécrété par une cellule souche mésenchymateuse comprenant au moins une propriété biologique d'une cellule souche mésenchymateuse, le procédé comprenant au moins une période de traitement des cellules avec une solution Ringer Lactate et/ou de la vitamine D.

2. Procédé selon la revendication 1, **caractérisé en ce que** le procédé comprend en outre au moins une période de maintien des cellules au moins 5 minutes dans des conditions anoxiques.

3. Procédé selon la revendication 2, **caractérisé en ce que** la période de maintien des cellules dans des conditions anoxiques est suivie d'une période de maintien des cellules dans des conditions oxiques.

4. Procédé selon la revendication 3, **caractérisé en ce qu'**un cycle comprenant une période de maintien des cellules dans des conditions anoxiques et une période consécutive de maintien des cellules dans des conditions oxiques est conduit plus d'une fois, de préférence 2 à 4 fois.

5. Procédé selon l'une quelconque des revendications 2 à 4, **caractérisé en ce que** la concentration d'oxygène pendant l'étape anoxique est inférieure à 1 %, de préférence inférieure à 0,5 %.

6. Procédé selon l'une quelconque des revendications 2 à 5, **caractérisé en ce que** des exosomes, des microvésicules et/ou des substances de sécrétome paracrine sont isolés séparément ou dans des groupes du sécrétome.

7. Procédé selon l'une quelconque des revendications 2 à 6, dans lequel les cellules sont maintenues dans des conditions anoxiques dans une fiole anaérobie, un incubateur sous CO₂/O₂ ou un autre dispositif pour cultiver des cellules dans des conditions anoxiques.
